# EUROPEAN PATENT APPLICATION

(11) **EP 3 090 742 A1**
(43) Date of publication of application: **09.11.2016**
(21) Application number: 14870518.9
(22) Date of filing: 11.12.2014
(51) Int. Cl.: A61K 31/353, A61K 36/185, A61P 1/04

(54) **USE OF COMPOUNDS PRODUCED FROM ARRABIDAEA BRACHYPODA EXTRACTS AS ANTI-ULCEROGENIC AGENTS**

(30) Priority: 12.12.2013 BR 13031926
(71) Applicant: Universidade Estadual Paulista "Julio De Mesquita", 01140-070 São Paulo, SP (BR); Universite De Geneve, 1211 Genève 4 (CH); Universidade Estadual de Campinas - Unicamp, 13083-970 Campinas - SP (BR)
(72) Inventor: QUINTINO DA ROCHA, Cláudia, CEP: 14801-970 Araraquara, SP (BR); VILEGAS, Wagner, CEP: 11330-900 São Vicente, SP (BR); FERREIRA QUEIROZ, Emerson, 1211 Genève 4 (CH); WOLFENDER, Jean-Luc, 1211 Genève 4 (CH); MARCOURT, Laurence, 1211 Genève 4 (CH); MONTEIRO SOUZA BRITO, Alba Regina, CEP: 13083-970 Campinas, SP (BR); MEIRA DE FARIA, Felipe, CEP: 13083-970 Campinas, SP (BR)
(74) Representative: Carvajal y Urquijo, Isabel
(86) International application number: PCT/BR2014/000439
(87) International publication number: WO 2015/085386

(57) **Abstract**

This invention discloses the use of compounds obtained from roots, stem, bark and leaves extracts from plant species of genus *Arrabidaea,* in particular *Arrabidaea brachypoda,* also known in Brazil under the names "cipó-una" or "cervejinha do campo", to treat ulcerogenic diseases.

## Description

### Field of the invention:

This invention discloses the use of compounds obtained from roots, stem, bark and leaves extracts from plant species of genus *Arrabidaea,* in particular *Arrabidaea brachypoda,* also known in Brazil under the names "cipó-una" or "cervejinha do campo", to treat ulcerogenic diseases.

### Background of the invention:

The denomination ulcer generically discloses any superficial injury on cutaneous or mucous tissue. In such injuries, the rupture of the epithelium occurs in a way to expose deeper tissues.

Ulcers can be classified according to the type of tissue attacked. Thus, cutaneous ulcers attack the skin (non-hairy areas or scalp), mucous ulcers attack mucous membranes (external or internal), serous ulcers attack serous membranes and complex ulcers attack different types of tissues along its extension or depth.

The peptic ulcer is an example of injury which occurs on the coating of the stomach or in the first part of the small intestine, called duodenum. When placed on the stomach, the peptic ulcer is denominated gastric ulcer.

The factors which hurt the gastric mucous originate from a variety of endogenous or exogenous stimuli, among which are the alcohol consumption, smoking, inappropriate diet, non-steroidal anti-inflammatories (NSAIs) consumption and the local presence of the bacteria *Helicobacter pylori.*

Among the therapeutic options existing on the market for treating ulcers, drugs which promote an antibacterial action, the neutralization of the gastric acid, the reduction of the stomach acid secretion and cytoprotection are available.

Proton pump inhibitors (such as omeprazol and lansoprazole) and antagonists of the histamine H₂ receptor (cimetidine) are used. Drugs such as carbenoxolone and indomethacin are also used. The drugs available predominantly operate by minimizing the harmful factors of the mucous tissue and the therapy is associated to the appearance of adverse effects and, in many cases, the recurrence of the gastric injury occurs.

In view of the difficulties pointed out above, on the prevention and on the treatment of the diseases, this invention proposes the use of the compounds obtained from *Arrabidaea brachypoda* in the treatment of ulcerogenic diseases.

### State of art:

Documents available on the state of art disclose different compounds obtained from *Arrabidaea,* as well as their incorporation in pharmaceutical compositions.

The Brazilian document PI 0600943-3 A2 discloses the use of phytoterapic extracts based on *Arrabidaea chica* as antifungal and antibacterial. The extracts can be used as a drug or a cosmetic additive, and can be directly applied or incorporated to a pharmacologic or cosmetic vehicle.

On document WO 2013/091056, it is disclosed stable pharmaceutical compositions comprising *Arrabidae chica* extract in controlled release systems, in the form of liposomes, microparticles or nanoparticles.

In WO 0152809, it is disclosed cosmetics or pharmaceutical preparations containing an active amount of *Arrabidae chica* extract, while in JP 2001/122763 it is disclosed a composition for external use comprising plants, such as those of the genus *Arrabidaea,* as well as their extracts.

None of the documents of the state of art discloses the use of compounds obtained from *Arrabidaea brachypoda* for treating ulcerogenic diseases.

### Summary of the invention:

This invention discloses the use of compounds obtained from *Arrabidaea brachypoda* extracts to treat ulcerogenic diseases.

### Brief description of the figures:

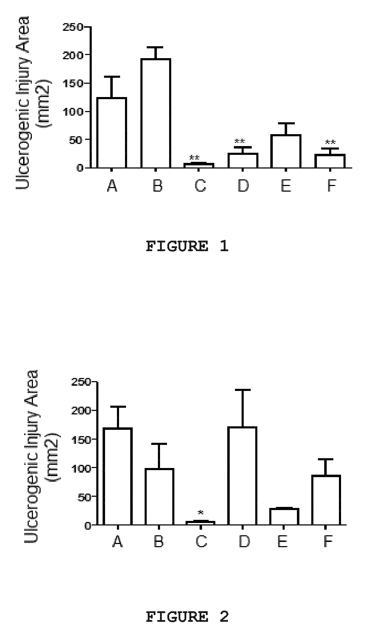

Figure 1 graphically represents the effect of the oral administration of the A. *brachypoda* roots extract in animals submitted to the model of ulcer induced by ethanol, wherein A represents the control (saline), B represents the control (saline) with pre-treatment with N-nitro-L-arginine methyl ester (L-NAME) on the area of the ulcerogenic injury, C represents the carbenoxolone, D represents the carbenoxolone with pre-treatment with N-nitro-L-arginine methyl ester (L-NAME) on the area of the ulcerogenic injury, E represents the A. *brachypoda* roots extract and F represents the same extract with pre-treatment with N-nitro-L-arginine methyl ester (L-NAME) on the area of the ulcerogenic injury.
Figure 2 graphically represents the effect of the oral administration of the A. *brachypoda* roots extract in animals submitted to the model of ulcer induced by ethanol, wherein A represents the control (saline), B represents the control (saline) with pre-treatment with N-ethylmaleimide (NEM) on the area of the ulcerogenic injury, C represents the carbenoxolone, D represents the carbenoxolone with pre-treatment with N-ethylmaleimide (NEM) on the area of the ulcerogenic injury, E represents the A. brachypoda roots extract and F represents the same extract with pre-treatment with N-ethylmaleimide (NEM) on the area of the ulcerogenic injury.
Figures 3 and 4 graphically represents the effect of the oral administration of the A. brachypoda roots extract for 7 and 14 consecutive days on the weight of the liver (A), lungs (B), testicles (C), heart (D) and kidneys (E) of animals submitted to the model of ulcer induced by acetic acid, wherein (a) is SHAM, (b) is saline, (c) is lansoprazole and (d) is the A. *brachypoda* extract.
Figure 5 graphically represents the evolution of the effect of the oral administration of the A. brachypoda roots extract for 14 consecutive days on the weight of the liver (A), lungs (B), testicles (C), heart (D) and kidneys (E) of animals submitted to the model of ulcer induced by acetic acid, wherein (a) is SHAM, (b) is saline, (c) is lansoprazole and (d) is the A. *brachypoda* extract.
Figures 6 and 7 graphically represents the effect of the oral administration of the A. brachypoda roots extract after 7 and 14 consecutive days on the healing of the injury induced by acetic acid in rats, by acetic acid, wherein (a) is saline, (B) is lansoprazole and (C) is *A. brachypoda.*
Figure 8 graphically represents the evolution of the healing process of the injury induced by acetic acid in rats orally treated with the A. brachypoda roots extract for 14 consecutive days, wherein (A) is saline, (B) is lansoprazole and (C) is A. *brachypoda.*
Figure 9 graphically represents a comparison of the enzymatic activity of MPO in animals treated with saline (A), lansoprazole (B) and *A. brachypoda* extract (C) for 7 (a) and 14 (b) days.
Figures 10A and 10B are photomicrographs of rats stomachs with ulcer induced by acetic acid after treatment with saline for 7 consecutive days, in magnifying lens of 20x (A) and 100x (B).
Figures 10C and 10D are photomicrographs of rats stomachs with ulcer induced by acetic acid after treatment with lansoprazole for 7 consecutive days, in magnifying lens of 20x (C) and 100x (D).
Figures 10E and 10F are photomicrographs of rats stomachs with ulcer induced by acetic acid after treatment with A. *brachypoda* for 7 consecutive days, in magnifying lens of 20x (E) and 100x (F).
Figures 11A and 11B are photomicrographs of rats stomachs with ulcer induced by acetic acid after treatment with saline for 14 consecutive days, in magnifying lens of 20x (A) and 100x (B).
Figures 11C and 11D are photomicrographs of rats stomachs with ulcer induced by acetic acid after treatment with lansoprazole for 14 consecutive days, in magnifying lens of 20x (C) and 100x (D).
Figures 11E and 11F are photomicrographs of rats stomachs with ulcer induced by acetic acid after treatment with A. *brachypoda* for 14 consecutive days, in magnifying lens of 20x (E) and 100x (F).
Figure 12 graphically represents the gastroprotection promoted by the oral administration of saline (A), lansoprazole (B) and A. brachypoda hydro alcoholic roots extract (10, 30, 100 and 300 mg.Kg-1, respectively C, D, E and F) in the model of ulcer induced by ethanol in rats.
Figure 13 graphically represents the effects of the oral pre-treatment of SHAM (A), saline (B), carbene (C) and A. brachypoda hydro alcoholic extract (D) in the model of ulcer induced by NSAIs.
Figure 14 graphically represents the effects of the oral administration of saline (A), carbene (B) and A. brachypoda hydro alcoholic extract (C) on the production of mucus adhered by the gastric mucous for rats submitted to pylorus ligature.

### Detailed description of the invention:

This invention discloses the use of compounds obtained from roots, stem, bark and leaves extracts from species of genus *Arrabidaea,* in particular from roots of *Arrabidaea brachypoda,* also known in Brazil under the names "cip6-una" or "cervejinha do campo".

The roots extract of this plant comprises fractions, which are enriched in compounds presenting an anti-ulcerogenic activity.

### Method of obtainment of the compounds:

The compounds of the present invention are obtained from patterned extracts of species of genus *Arrabidaea,* in particular *Arrabidaea brachypoda.*

For obtaining the extract, the biomass comprising roots, stem, bark and leaves, as well as mixtures thereof, is extracted by percolation, soxhlet maceration or by gases in supercritical state, as well as a mixture of those techniques.

The biomass can be green and / or dry, pulverized, milled, in pieces and / or in crumbs. Optionally, the biomass is frozen before being processed.

The previous freezing of the plant material avoids the thermic degradation provoked by the heating through the electric mill.

The extraction is made by using basic or acid medium, in aqueous medium or using organic solvents, isolated or in combination.

The acids can be strong or weak, diluted or concentrated, isolated or in mixtures.

In specific embodiments of the invention, the acid is selected from acetic acid, hydrochloric acid and formic acid.

The bases, in turn, can be concentrated or diluted, isolated or in mixtures.

In specific embodiments of the invention, the base is selected from ammonium hydroxide (NH₄OH) and sodium carbonate (Na₂CO₃).

The organic solvent is selected among those which comprises halogenated compounds, alcohols, aldehydes, ethers, esters, ketones, alkanes, cycloalkanes, phenolic compounds, benzenes and derivatives, isolated or its mixtures.

The extract obtained can be dry with the spray drying technology, under reduced pressure or at room temperature.

For the spray drying technique, the input and output temperatures vary from 150 and 190°C and 80 and 90°C, respectively. For the drying under reduced pressure, it varies between 25 and 100°C.

The different compounds are isolated and purified through chromatographic techniques, under pressure or not, such as chromatography under atmospheric pressure or chromatography under low, medium or high pressure.

To perform the technique, a regular stationary phase can be used, such as silica gel, or a reversed phase, such as C-8 or C-18, can be used.

Furthermore, it can be used a liquid/liquid partition, such as counter flow chromatography, or a centrifuge partition, by using ion-exchange resins or filtration membranes.

With a view of identifying the molecules responsible by the pharmacologic activity, the *Arrabidaea brachypoda* roots extract is submitted to a guided fractioning.

The extract is fractioned through chromatography under medium pressure (MPLC) in reversed phase, with a solvent system having methanol-water gradients which rates from 10:90, with a gradual increase up to 100%.

The highest pressure is of 20 bar (2x10³ kPa) and the samples are allotted in an input cartridge after its mixture with the stationary phase (silica) in a proportion of 1:3 by weight.

The fractions are evaluated on the anti-ulcerogenic tests. From such fractions, the isolated compounds RAB-01, RAB-02 and RAB-03 are obtained, which structures are elucidated through spectroscopic methods (such as ultraviolet - UV, nuclear magnetic resonance - NMR 1D and 2D, mass spectrometry in low and high resolution - MS and HRMS), apart from chemical and enzymatic reactions.

The isolated compounds were identified as dimeric flavonoids of the formula I below: 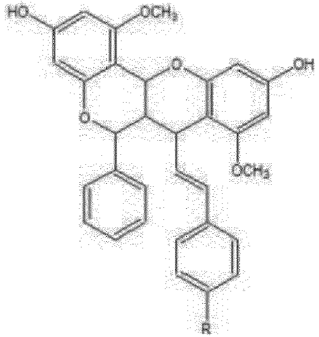 wherein, to RAB-01, R is hydroxyl; to RAB-02, R is methoxyl and to RAB-03, R e hydrogen.

Their structure is identified through classical structural elucidation techniques, including ultraviolet, nuclear magnetic resonance (NMR) and mass spectrometry (MS).

The data related to hydrogen nuclear magnetic resonance (MeOD, 500 MHz), carbon nuclear magnetic resonance (MeOD, 125 MHz) and mass spectrometry for each one of the components RAB-01, RAB-02 and RAB-03 are disclosed below and in tables 1, 2 and 3:
**RAB-001** - Red amorphous solid; [α]_{D}^{20,1} -108,43° (c 0.1 MeOH) ; UV λₘₐₓ (log □) 266 nm (5.02); ¹H-NMR (CD₃OD, 500 MHz) e ¹³C-NMR (CD₃OD, 125 MHz): Table 1; ESI-MS (positive mode): *m*/*z* 525.1 [M+H]⁺; HRMS: *m*/*z* 525.1926 [M+H]⁺ (calculated for C₃₂H₂₈O₇, 525.1913, Δ ppm = 1.3).
**RAB-02** - Orange amorphous solid; [α]_{D}^{20,9} -118, 80° (c 0.1 MeOH) ; UV λₘₐₓ (log □) 254 nm (4.82); ¹H-NMR (CD₃OD, 500 MHz) e ¹³C-NMR (CD₃OD, 125 MHz): Table 1; ESI-MS (positive mode) *m*/*z* 539.1 [M+H]⁺; HRMS (positive mode) *m*/*z* 539. 2095 [M+H]⁺ ]⁺ (calculated for C₃₃H₃₀O₇, 539. 2070, Δ ppm = 4.6).
**RAB-03** - Orange amorphous solid; [α]_{D}^{20,1} -82,86 (c 0.1 MeOH) ; UV λₘₐₓ (log □) 266 nm (5.78); ¹H-NMR (CD₃OD, 500 MHz) e ¹³C-NMR (MeOD, 125 MHz): table 1; ESI-MS (positive mode) *m*/*z* 509.1 [M+H]⁺; HRMS (Pos. Mod): 509.1974 [M+H]⁺ ]⁺ (calculated for C₃₂H₂₉O₆, 509.1964, Δ ppm = 2.0).

**Table 1. ¹H NMR and ¹³C NMR data (500 and 125 MHz, CD₃OD, δ in ppm) of compound RAB-01.**

| **No.** | **¹H** | **¹³C** |
|---|---|---|
| **1** | | 161.1 |
| **2** | 6.08 d, *J* = 2.2 | 92.8 |
| **3** | | 161.1 |
| **4** | 5.94 d, *J* = 2.2 | 96.4 |
| **4a** | | 157.8 |
| **6** | 4.78 d, *J* = 11.2 | 78.2 |
| **6a** | 2.22 dt, J = 11.2, 1.9 Hz | 42.4 |
| **7** | 3.17 ddd, J = 5.5, 1.9, 1.6 Hz | 34 |
| **7a** | | 101.8 |
| **8** | | 160.2 |
| **9** | 6.05 d, J = 2.1 Hz | 93 |
| 10 | | 159.1 |
| 11 | 5.98 d, J = 2.1 Hz | 96.5 |
| **11a** | | 155.4 |
| **12a** | 5.28 d, J = 2.3 Hz | 63.5 |
| 12b | | 103.8 |
| **1'** | | 140.4 |
| **2' ,6'** | 7.24 d, J = 7.6 Hz | 128.5 |
| **3',5'** | 7.38 m | 129.5 |
| **4'** | 7.38 m | 129.6 |
| **A** | 5.95 dd, *J* = 15.9, 5.5 Hz | 130.7 |
| **B** | 5.81 dd, J = 15.9, 1.6 Hz | 131.7 |
| **1"** | | 130.4 |
| **2", 6"** | 7.05 d, J = 8.6 Hz | 128.4 |
| **3", 5"** | 6.64 d, J = 8.6 Hz | 116.2 |
| **4"** | | 157.7 |
| **1-OMe** | 3.81 s | 56.1 |
| **8-OMe** | 3.62 s | 55.9 |
| **4'-OMe** | - | - |

**Table 2. ¹H NMR and ¹³C NMR data (500 and 125 MHz, CD₃OD, δ in ppm) of compound RAB-02.**

| **No.** | **¹H** | **¹³C** |
|---|---|---|
| **1** | | 160.9 |
| **2** | 6.08 d, *J* = 2.1 Hz | 92.7 |
| **3** | | 161,1 |
| **4** | 5.94 d, *J* = 2.1 Hz | 96.3 |
| **4a** | | 157.7 |
| **6** | 4.77 d, *J* = 11.3 Hz | 78.1 |
| **6a** | 2.19 dt, *J* = 11.3, 1.9 Hz | 42.3 |
| **7** | 3.17 ddd, *J* = 5.6, 1.9, 1.6 Hz | 34 |
| **7a** | | 101.6 |
| **8** | | 160.2 |
| **9** | 6.05 d, J = 2.1 Hz | 93 |
| **10** | | 159.2 |
| **11** | 5.98 d, *J* = 2.1 Hz | 96.5 |
| **11a** | | 155.3 |
| **12a** | 5.27 d, *J* = 2.3 Hz | 63.4 |
| **12b** | | 103.7 |
| **1'** | | 140.4 |
| **2',6'** | 7.23 d, *J* = 7.0 Hz | 128.5 |
| **3',5'** | 7.38 m | 129.5 |
| **4'** | 7.38 m | 129.5 |
| **A** | 5.99 dd, *J* = 15.8, 5.6 Hz | 131.5 |
| **B** | 5.83 dd, *J* = 15.8, 1.6 Hz | 131.3 |
| **1"** | | 131.1 |
| **2", 6"** | 7.11 d, *J* = 8.8 Hz | 128.2 |
| **3", 5"** | 6.75 d, *J* = 8.8 Hz | 114.8 |
| **4"** | | 160.2 |
| **1-OMe** | 3.79 s | 56.1 |
| **8-OMe** | 3.62 s | 55.9 |
| **4'-OMe** | 3.73 s | 55.6 |

**Table 3. ¹H NMR and ¹³C NMR data (500 and 125 MHz, CD₃OD, δ in ppm) of compound RAB-03.**

| **No.** | **¹H** | **¹³C** |
|---|---|---|
| 1 | | 161.1 |
| 2 | 6.08 d, *J* = 2.2 Hz | 92.8 |
| 3 | | 161.1 |
| 4 | 5.95 d, *J* = 2.2 Hz | 96.4 |
| 4a | | 157.8 |
| 6 | 4.79 d, *J =* 11.3 Hz | 78.2 |
| 6a | 2.24 dt, J = 11.3, 1.9 Hz | 42.2 |
| 7 | 3.21 ddd, J = 5.7, 1.9, 1.7 Hz | 34.2 |
| 7a | | 101.5 |
| 8 | | 160.2 |
| 9 | 6.05 d, J = 2.3 Hz | 93 |
| 10 | | 159.2 |
| 11 | 5.99 d, *J* = 2.3 Hz | 96.6 |
| 11a | | 155.4 |
| 12a | 5.28 d, J = 2.4 Hz | 63.5 |
| 12b | | 103.8 |
| 1' | | 140.4 |
| 2',6' | 7.24 d, *J =* 7.6 Hz | 128.5 |
| 3',5' | 7.38 m | 129.5 |
| 4' | 7.38 m | 129.6 |
| A | 6.15 dd, *J* = 15.7, 5.7 Hz | 133.8 |
| B | 5.91 dd, *J* = 15.7, 1.7 Hz | 132 |
| 1" | | 138.6 |
| 2", 6" | 7.18 m | 127.2 |
| 3", 5" | 7.18 m | 129.4 |
| 4" | 7.10 m | 128 |
| 1-OMe | 3.79 s | 56.1 |
| 8-OMe | 3.61 s | 55.9 |
| 4"-OMe | - | - |

### Incorporation of compounds RAB-01, RAB-02 and RAB-03 in pharmaceutical compositions:

The pharmaceutical compositions obtained according to the present invention comprise the compounds RAB-01, RAB-02 and RAB-03 in patterned amounts, in its isolated form or in association with other natural or synthetic products, in different proportions.

Preferably, the pharmaceutical compositions of this invention comprises the compounds RAB-01, RAB-02 and RAB-03 in a concentration range which varies from 0.001 to 50 % of at least one of the compounds in its free form or in a salt form (such as chlorates, sulphates or borates), associated to pharmaceutically acceptable excipients.

The pharmaceutical compositions can further comprise drugs, vitamins, salts and / or sugars.

The compositions herein disclosed are presented in the form of tablets, gelatin capsules (hard or soft), microcapsules, nanoparticles, pigments, syrups, emulsions of the type oil-in-water and water-in-oil, liposomes, injectable liquids, aerosols, powders, freeze-dried and similar, to be administered via oral, topic, injectable or inhalable.

The referred compositions are useful in the treatment of ulcers, in particular peptic ulcers.

### Examples of the invention:

As follows, expository examples of particular achievements of the present invention are presented, without creating any limitation to its scope.

### 1 - Obtainment of the Arrabidaea brachypoda hydro alcoholic extract:

In the bowl of an extractor endowed with mechanical stirring, it is added 1.5 kg of roots, stems or leaves of plants of genus *Arrabidaea,* such as *Arrabidaea brachypoda,* dried in stoves under controlled temperature at 60°C.

The material is pulverized and frozen in liquid nitrogen and, then, milled in an electric mill.

In turn, 9.0 L of ethanol 70% (v/v) was added, with constant stirring, for at least 150 hours.

The extraction tank is gradually heated, in a heating speed of 10 °C per minute, until a temperature between 45 and 50°C is achieved. Following, the extract is filtered while warm, in a temperature which varies from 35 to 40 °C at vacuum, through filters from 100 to 150 mm.

After the evaporation of the solvent in a rotative evaporator under reduced pressure and highest temperature of 40 °C, about 20g of a concentrated hydro alcoholic extract is obtained.

### 2 - Obtainment of the Arrabidaea brachypoda dichloromethane partition:

About 8.0 g of the hydro alcoholic extract is dissolved in 500 mL of a water / methanol 8:2 solution and 250 mL of dichloromethane in a temperature which varies from 20 to 25°C.

The solution is added to a separation flask and, after stirring, the phase separation is awaited protected from the light. The dichloromethanic phase is withdrawn and more 250 mL of dichloromethane is added, repeating the steps of stirring and phase separation protected from light.

The process is repeated until exhaustion, i.e., until the dichloromethanic phase no longer presents coloration when mixed with the aqueous phase. The dichloromethanic phase e evaporated in rotative evaporator, presenting a yield of 31.2%.

### 3 - Obtainment of the active compounds from the purification of the Arrabidaea brachypoda extract:

After obtainment of the hydro alcoholic extract (as shown in example 1) and obtainment of the dichloromethane partition (as shown in example 2), a dichloromethanic phase presenting a yield of 33.7% is obtained.

The dichloromethanic extract thus obtained is purified through medium pressure liquid chromatography (MPLC) using an eluent system composed by a mixture of methanol and water in gradient mode (5 % to 100 % in 72 hours) and a stationary phase to reversed phase (C-18 and 15-25 µm) in a column of 460 mm of width for 70 mm of diameter.

The compounds were detected by ultraviolet in 254 nm. The fractions obtained are submitted to several chromatographic techniques, in a way to isolate the compounds RAB-01, RAB-02 and RAB-03.

### Evaluation of the gastroprotective activity:

In a comparative manner, the drugs used to determine the anti-ulcerogenic activity of the compounds obtained from *Arrabidaea brachypoda* were:
- lansoprazole;
- carbenoxolone;
- indomethacin;
- cimetidine.

All drugs were immediately prepared before administration in saline solution with the concentration of 0.09%.

The animals used were male unib-WH rats, with weight from 150 to 250 grams. The animals were acclimated to the laboratory conditions for at least seven days before experimental manipulation, with controlled temperature (23 ± 2°C) and light-dark cycles of 12 hours. Furthermore, the same were fed with animal feed and water *ad libitum* and distributed by chance on the different experimental groups, being submitted to fasting (with water *ad libitum)* of at least 12 hours, depending on the trial type.

*- Role of the NO in gastroprotection (technique developed by Sikiríc* *et al. in* 1997):

The rats submitted to fasting for 24 hours were divided into groups according to the treatments.

The control group received a peritoneal injection of saline solution or the administration of L-NAME (N-nitro-L-arginine methyl ester), an inhibitor of NO-sintase by the same path (10 mg/kg).

After 30 minutes, the groups received (i) vehicle (saline), (ii) *Arrabidaea brachypoda* extract and (iii) carbenoxolone (positive control) orally.

Following, after 50 minutes of the administration of the pre-treatments, the animals orally received absolute ethanol. The animals were sacrificed after 1 hour and the stomachs were removed and opened toward the great curvature for determining the injury area.

### - Role of the sulfhydryl groups in gastroprotection (technique developed by Matsuda et al. in 1999):

After a 24-hour fasting, the animals were divided by chance into groups, treated with a subcutaneous injection of saline solution (negative control) or 10 mg/kg of N-ethylmaleimide (NEM), a blocker of the sulfhydryl groups.

After 30 minutes of the pre-treatment, the groups received vehicle (10 mL/kg), carbenoxolone (positive control) and *A*. *brachypoda* extract (300mg/kg) orally.

Thirty minutes after the treatment, all animal groups received absolute ethanol orally. One hour later, the animals were sacrificed and the stomachs were removed and opened toward the great curvature for determining the injury area.

### - Measurement of the PGE₂ levels (technique developed by Curtis et al. in 1995):

The tissue of the animals submitted to ulcer induced by NSAIs was homogenized and prepared according to an already known method, followed by sensitization and reading ELISA.

### Dosages in animal tissues submitted to ulcer induced by ethanol:

### - Measurement of the glutathione levels (GSH):

To proceed with the measurement of the total glutathione content, the method disclosed by Anderson in 1985 was used, which bases on the total oxidation of the reduced glutathione (GSH) present in a sample to its oxidized form (GSSG), through the incubation of the sample with dithiobis nitrobenzoic acid (DTNB).

The reduced DTNB acquires a yellow coloration, which can be spectrophotometrically determined. The GSSG generated is reduced by the action of the enzyme glutathione redutase in the presence of NADPH.

The GSH formed oxidizes again, generating a continuous cycle, in which the reduction speed of the DTNB (and its resulting increase in the absorbance at 412 nm) is proportional to the total amount of glutathione (GSH+GSSG).

To proceed with the measurement of the total glutathione content, the cytosolic extracts obtained from the stomachs of the animals of the ulcer induced by ethanol trials were used.

The samples, after defrosting, had 20 µL of its volume withdrawn and pipetted in a plate comprising 96 holes, wherein 140 µL of NADPH was added, along with 5 µL of PBS and 20 µL of DTNB.

The plate was placed on the spectrophotometer reader, where it was maintained for 5 minutes in a temperature of 30 °C.

After this period, 15µL of glutathione redutase was added and the increase on the absorbance was registered at 412 nm. The results were expressed as nmol/g of tissue.

### - Measurement of the glutathione redutase (GR) activi ty:

For measuring the GR activity, the enzymatic reaction was prepared with 100 µL of the sample (after centrifugation and dilution of the supernatant in phosphate buffer pH = 7.4 in a 1:10 ratio) and 150 µL of the mixture formed by EDTA (0.20 mM), oxidized glutathione (1 mM) and NADPH (0.1 mM). The absorbance was read at 365 nm, between 0 and 10 minutes, according to the technique developed by Carlberg & Mannervik, 1985.

### - Measurement of the glutathione peroxidase (GPx) activi ty:

The samples were centrifuged at 12000 rpm, at 4°C, for 15 minutes and the supernatant was diluted in phosphate buffer 0,1 M (pH = 7,4) in a 1:10 ratio.

In an aliquot of 100 µL of the scraped material, 150 µL of reduced glutathione (10 mM), NADPH (4 mM) and glutathione redutase (1U) and 20 µL of H₂O₂ (25 mM) was added, according to the method developed by Yoshikawa et al. in 1993. The absorbance was determined at 365 nm, between 0 and 10 minutes.

### - Evaluation of the healing effect of the ulcer induced by acetic acid (technique developed by Takagi et al. in 1969):

The rats were anaesthetized with xilazine chloridrate and ketamine for performing a longitudinal incision right under the xiphoid apophysis.

The previous wall of the stomach was exposed and an area of the serous tissue was delimited with a plastic ring upon which was added 50 µL of acetic acid absolute with the aid of an automatic micropipette, the contact between the acid and the serous tissue being maintained during 60 seconds.

Following, the area was cleaned with saline solution and the animals had their abdominal cavities closed, being maintained in rest until complete recovery of the anesthesia and, then, they were placed at the vivarium.

After 48 hours of the surgical procedure, the oral administration of the treatments was initiated (once daily for 7 and 14 days) with EAB (300 mg / kg), lansoprazole (30 mg / kg - positive control) and vehicle (saline solution 0.9% - negative control).

The animals were weighted and observed from the induction of the injury to the end of treatments. At the end of this period, the animals were sacrificed; their stomachs were removed and opened toward the great curvature to the measurement of the ulcerative injury area.

Tissue sections were also collected for preparing histological blades. The animals also had heart, lungs, liver, kidneys and testicles collected and weighted in order to verify possible signs of toxicity.

### - Expression of the proteins COX-1, COX-2 and EGF:

The gastric homogenate was centrifuged at 12000 rpm at 4 °C for 15 minutes. Determined protein values (50 ug) were applied on a polyacrylamide gel and submitted to electrophoresis, with buffer solution. The gel was subjected to 120V during the whole process.

Following, the proteins separated on SDS-PAGE were transferred to a nitrocellulose membrane in and electrotransfer equipment, with the membranes soaked in transfer buffer, maintained in a constant voltage of 25V, current 1.0 A for 12 minutes.

The nitrocellulose membranes containing the transferred proteins were incubated in blocking solution for one hour to reduce nonspecific binding proteins.

Following, the membranes were subjected to successive washings with buffer, in 5-minute intervals. The membrane was incubated at 4 °C overnight, using specific antibodies for COX-1, COX-2 and EGF.

After this procedure, the nitrocellulose membrane was subjected again to successive washes with buffer and, then, incubated at room temperature for 1 hour with secondary antibodies for COX-1, COX-2 and EGF.

To detect immunoreactive bands, the membranes were soaked in chemiluminescent solution for 5 minutes. After this period, the membranes were placed in a fotodocumenter for detecting the fluorescent bands and later densitometry analysis with the aid of a specific software.

### - Measurement of the MPO activity:

Since it relates to an important inflammatory indicator, the MPO activity was also verified in the model of chronic ulcer.

### - Preparation of the histological blades:

The histological cuts and blades were prepared by fixing the material in Alfac (formalin, alcohol 80% and acetic acid) for 24 hours. The pieces were, then, dehydrated and included in an appropriate medium solid.

Subsequently, the middle blocks were cut (7 mm thick) on a microtome, serially. The samples were submitted to hematoxylin-eosin (HE) coloration for general observation of the structure and cellularity in light microscopy.

### - Statistical analysis:

All the results were statistically treated and expressed as an average ± standard deviation or error and submitted to an one way analysis of variance and, subsequently, Dunnett and / or Tuckey test with a minimal significance level of *P < 0,05.

### - Gastric ulcer induced by ethanol (technique developed by Morimoto et al. in 1991):

Rats Unib-WH were submitted to fasting for 24 hours with water *ad libitum.* On the day of the trial, they were divided into 7 groups: saline as negative control, lansoprazole as positive control and four groups with the *A. brachypoda* roots extracts at different concentrations (dosages of 10, 30, 100 and 300 mg.Kg-1) dissolved in saline at 10 mL.Kg-1 concentrations.

The animals received the predicted treatment for each group and one hour later, 1 mL of absolute ethanol was orally administered.

After one hour of ulcer induction, the rats were sacrificed by cervical dislocation and their stomachs were withdrawn for quantification of injuries.

The injury areas were calculated, the stomachs were homogenized, the cytosolic extracts were prepared and the membranes for conducting the biochemical assays.

### - Gastric ulcer induced by non-steroidal anti-inflammatory (NSAIs) :

The animals were submitted to fasting for 24 hours with water *ad libitum.* On the day of the trial, the animals were divided into five groups: SHAM, saline, carbenoxolone, treatment with A. *brachypoda* extract to be submitted along with the administration of indomethacin and treatment with the plant extract where the indomethacin is not administered, for further evaluation of the prostaglandin E2 levels.

The groups received their treatments orally and, after 30 minutes, they were given the harmful agent, indomethacin (50 mg.Kg-1 solubilized in sodium carbonate 0.5%) in all groups except the SHAM group and the group which received plant treatment just for PGE verification.

Elapsed six hours from the indomethacin administration, all animals were sacrificed by cervical dislocation and they had their stomachs withdrawn for counting injuries. The glandular portion was scraped off and frozen at -80 °C for biochemical assays.

### - Pylorus ligature and evaluation of the parameters of the gastric juice (technique developed by Shay and Gruenstein in 1946):

The rats were in fasting for 48 hours with water ad *libitum.* On the day of the trial, the animals were divided into four groups: saline, lansoprazole, cimetidine and treated with A. *brachypoda* extract.

The rats were anesthetized and underwent a longitudinal incision just below the xiphoid apophysis for the pylorus ligature.

The drug administration was carried out immediately after the ligature, intraduodenally. The incisions were sutured, and after four hours of surgery, the rats were sacrificed. The stomachs were carefully withdrawn and acid secretion was collected. After centrifugation, the volume and pH of the secretion of each animal was measured.

### - Measurement of the production of mucus adhered to the gastric mucous (technique developed by Corne et al. in 1974) :

The rats were in fasting for 24 hours with water ad *libitum* for the performance of the trial. The animals were divided into three groups: saline, carbenoxolone and A. *brachypoda* extract.

The groups received the respective treatments orally and, after 1 hour, the pylorus ligature was performed. After four hours, the animals were sacrificed and the stomachs were withdrawn and opened toward the great curvature.

The glandular segments of the stomach were removed and weighted and each segment was transferred immediately to a tube comprising a pigment agent. After immerging for 2 hours in this solution, the pigment excess was removed through two successive washes with 10 mL of sucrose solution 0.25 M, first for 15 minutes and, then, for 45 minutes.

The pigment complexed with the mucus of the gastric wall was extracted with 10 mL of MgCl₂ 0,5 M by intermittent stirring for 1 minute, at 30 minutes intervals, during 2 hours.

An aliquot of 4 mL of the blue extract was vigorously stirred with an equal diethilic ether volume. A resulting emulsion was centrifuged at 3600 rpm for 20 minutes and the absorbance of the aqueous layer was determined at 580 nm.

The amount of pigment extracted per gram of glandular tissue was, then, calculated and expressed as pigment concentration in µg per mL of the solution per gram of the glandular portion.

### - Myeloperoxidase (MPO) activity:

The myeloperoxidase activity was determined through the method disclosed by Krawisz et al. in 1984. The samples were homogenized in hexadecyl trimethyl ammonium bromide (HTAB) buffer at 0,5% (w/v) in saline phosphate buffer (50 mM, pH 6,0) with final dilution of 1:20 (w/v) until the obtainment of an uniform aspect.

The HTAB acts like a detergent, making it easy to free the MPO enzyme from the azurofiles granules of the neutrophils, where it is stored.

The homogenate was submitted to the ultrasonic bath for 10 seconds, followed by a triple process for freezing and defrosting, which facilitates the rupture of the cell structures and, as a consequence, the release of the enzyme.

The sample was centrifuged at 7000 G, during 10 minutes and the MPO activity was determined following the kinetics of the reaction faced to hydrogen peroxide.

In an ELISA plate, 50 µL of the supernatant and 150 µL of the color reactive and hydrogen peroxide (0.066%) were placed in phosphate buffer (50mM, pH 6.0).

The absorbance was determined at 450 nm and the enzyme activity was calculated by the interpolation of the standard curve, performed with MPO originated from human neutrophils.

One unity of MPO is defined as the necessary amount to degrade 1 mmol/minute of hydrogen peroxide at 25° C. The results were expressed as U/g of protein.

### Results obtained:

### - Role of the NO in gastroprotection:

Figure 1 shows the data obtained on the evaluation of the role of the NO in gastroprotection promoted by A. *brachypoda* (300 mg.kg⁻¹).

The results shown indicate that the gastroprotection promoted by the A. *brachypoda* extract may not be mediated by NO, because, even with its production being blocked by the L-NAME, the activity was verified by the reduction of the damage to the gastric mucous, for the animals which did not received L-NAME and for the animals which did receive it.

### - Role of the sulfhydryl groups in gastroprotection:

In Figure 2, it is graphically represented the results of the observation made on the role of the sulfhydryl groups in the protection to the gastric mucous promoted by the oral administration of A. *brachypoda (300 mg.kg⁻¹).*

The results obtained demonstrate that the saline group associated to NEM (B) did not respond to the pre-treatment with NEM. However, an analysis of the result with the groups treated with carbenoxolone allows to suggest that A. *brachypoda* may not depend on the sulfhydryl groups to promote protection to the gastric mucous, even though such experimental model has presented abnormal variation on the negative control.

### - Antioxidant activity trials:

The trials evaluated the antioxidant activity of the enzymes glutathione redutase (GR), glutathione peroxidase (GPx) and the levels of sulfhydryl groups (GSH) on the gastric mucous of rats submitted to the model of ulcer induced by ethanol.

**Table 1 - Effect of the oral administration of the A. brachypoda roots extract on the enzymatic activity of GR and GPx on the gastric mucous of rats submitted to the model of ulcer induced by ethanol.**

| **Treatments** | **GR (nmol/min/mg of protein)** | **GPx (nmol/min/mg of protein)** |
|---|---|---|
| **Saline** | 7.65 ± 0.37 | 19.39 ± 3.86 |
| **Lansoprazole** | 10.53 ± 0.42* | 33.54 ± 2.12* |
| ***A. brachypoda*** | 8.29 ± 0.85 | 21.91 ± 3.28 |

The data showed in Table 1 indicate that A. *brachypoda* does not pursue an action on the enzymes GR and GPx, contrary to lansoprazole which presented greater activity of those two enzymes on the gastric mucous of rats.

**Table 2 - Effect of the oral administration of the A. brachypoda roots extract on the GSH levels on the gastric mucous of rats submitted to the model of ulcer induced by ethanol.**

| **Treatments** | **GSH (nmol/mg of protein)** |
|---|---|
| **Saline** | 4.81 ± 1.15 |
| **Lansoprazole** | 13.45 ± 2.25* |
| ***A. Brachypoda*** | 7.55 ± 2.09 |

The GSH levels were not maintained or increased by the oral administration of *A*. *brachypoda* to the rats submitted to the model of ulcer induced by ethanol, since the treatment with lansoprazole led to the conservation or to the increase of the cytosolic level of GSH.

### - Ulcer induced by acetic acid - sub-chronic model: Sub-chronic toxicity evaluation:

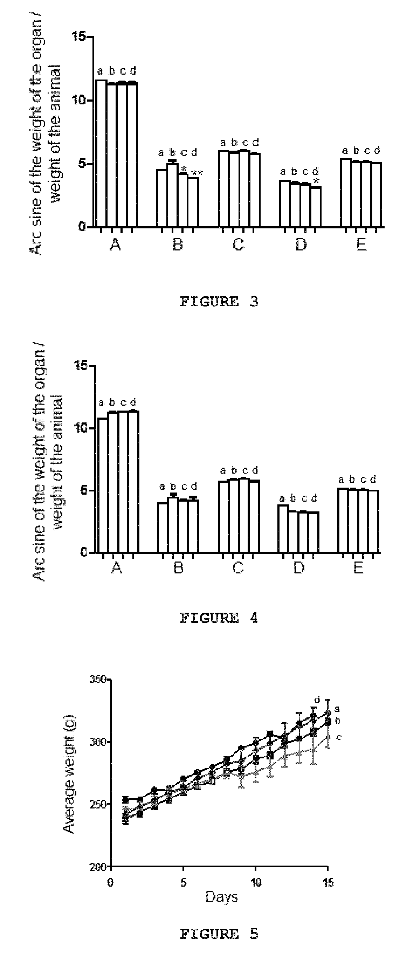

Figures 3, 4 and 5 are illustrative of the data which represent indicatives of sub-chronic toxicity of the experimental treatments.

An analysis of the relation between the weight of the organs and the animals treated for 7 days showed that the groups in which the lansoprazole and A. *brachypoda* were administered presented a reduction of this relation in the lungs, the last treatment also presented the same effect in the heart (Figure 3).

Figure 4 shows the same relation noticed on Figure 3, however, the analysis occurred after 14 days of treatment and significative differences were not observed.

Figure 5 shows the evolution of the corporal weight of the animals sub-chronically treated and also suits as a toxicity indicative. Even though it has occurred a decrease on the average corporal weight of the animals treated with lansoprazole, no significative statistical difference was observed.

### Injury healing:

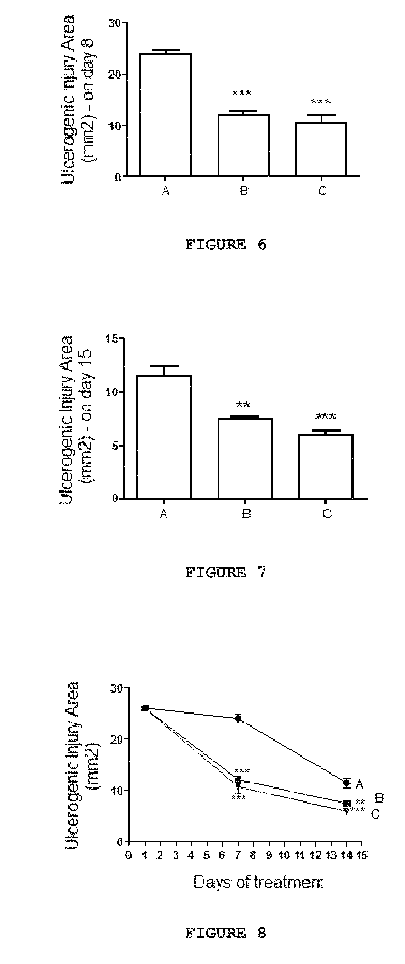

Figures 6 and 7 represent the data obtained on the inspection of the ulcerogenic injury area measured after 7 and 14 consecutive days of treatment.

On Figure 6, it is observed that the treatment with A. *brachypoda* promoted the reduction of the injury area after the period of 7 days, as well as the control group administered with lansoprazole.

According to Figure 7, it is clear that the treatment with A. *brachypoda* maintains its healing effect after 14 consecutive days of treatment, being more efficient than the lansoprazole control group.

Figure 8 shows the evolution of the healing process, evinced by the reduction of the injury area during the treatment period. It is important to emphasize that, after 20 days, the injuries are physiologically recovered.

The data shown evince an acceleration promoted by the treatments with lansoprazole and A. *brachypoda* in the recovery of the gastric mucous in rats.

### - MPO activity:

Tables 3 and 4 rejoin the data obtained on the evaluation of the enzymatic activity of MPO enzyme.

**Table 3. Effect of the oral administration of the A. brachypoda roots extract for 7 consecutive days on the MPO activity of the gastric mucous in animals submitted to the model of ulcer induced by acetic acid.**

| **Treatments** | **MPO (U/mg of protein)** |
|---|---|
| **Saline** | 125.10 ± 41.62 |
| **Lansoprazole** | 110.50 ± 33.43 |
| *A. **brachypoda*** | 166.00 ± 21.61 |

After 7 days of treatment, the experimental groups presented high MPO enzyme activity. Further, the group treated with A. *brachypoda* presented higher values than the other groups, even though it was not verified a statistical difference.

**Table 4. Effect of the oral administration of the A. brachypoda roots extract for 14 consecutive days on the MPO activity of the gastric mucous in animals submitted to the model of ulcer induced by acetic acid.**

| **Treatments** | **MPO (U/mg of protein)** |
|---|---|
| **Saline** | 148.60 ± 40.30 |
| **Lansoprazole** | 98.05 ± 15.99* |
| *A. **brachypoda*** | 64.74 ± 22.02** |

The groups treated with lansoprazole and *A*. *brachypoda* for 14 consecutive days had a reduction of the MPO activity, with greater significance for the last one.

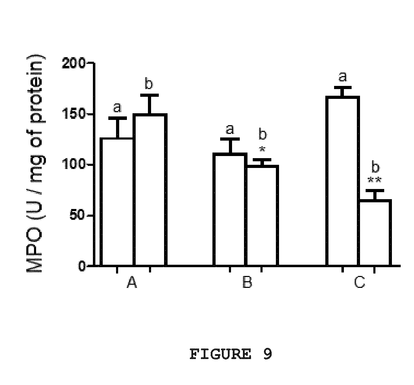

Figure 9 graphically joins the results of Tables 3 and 4, comparing them. An analysis of the results obtained for the MPO activity shows that it is high after 7 days of treatment, being reduced on the groups treated with lansoprazole and A. *brachypoda* for more than 7 days, suggesting an important role of the enzyme on the healing process.

### - Histological analysis:

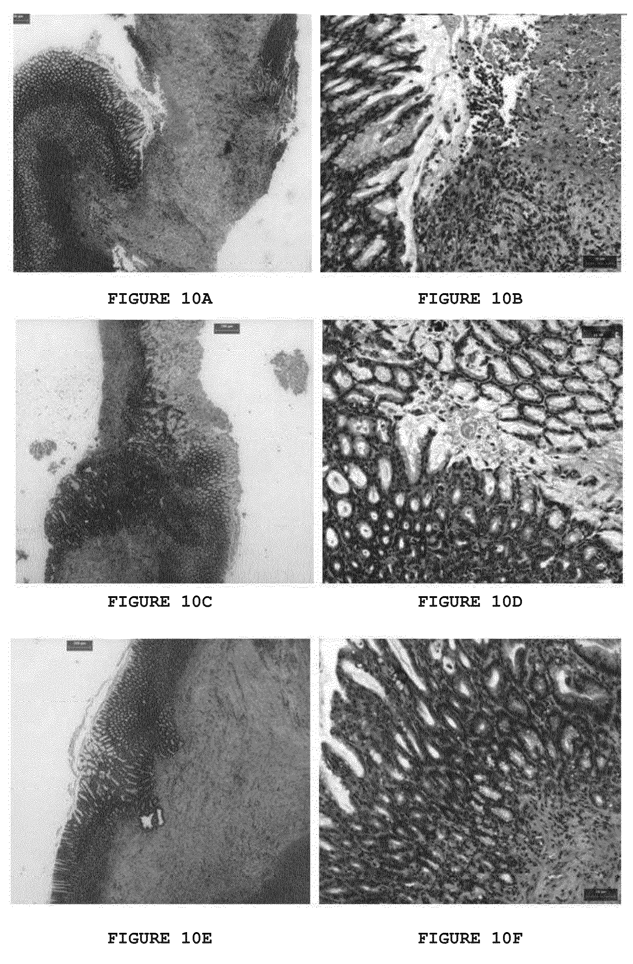

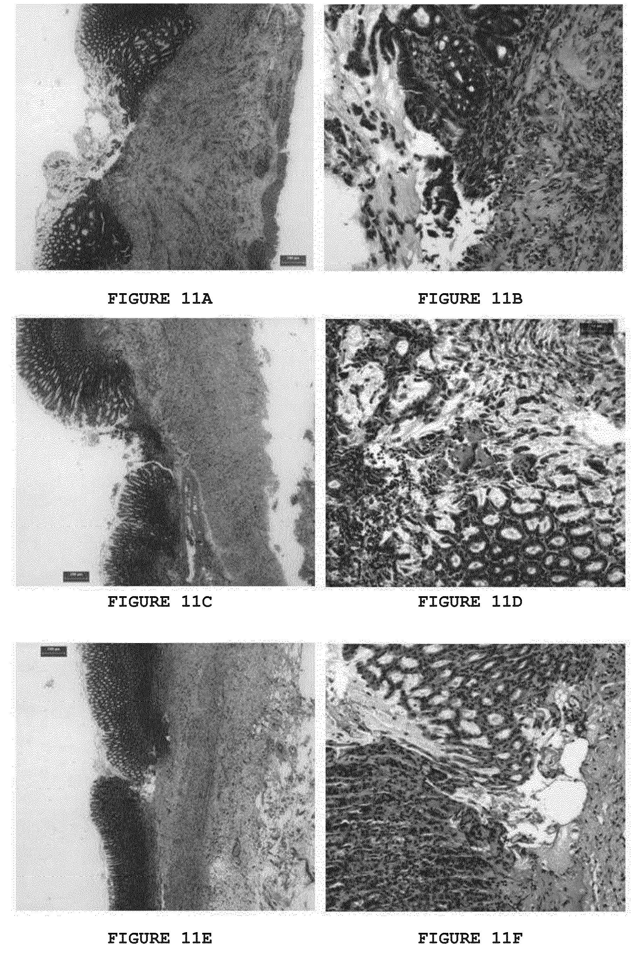

Figures 10A, 10B, 10C, 10D, 10E and 10F and 11A, 11B, 11C, 11D, 11E and 11F correspond to photomicrographs of the stomachs of the animals submitted to the sub-chronic model of ulcer induced by acetic acid and treated for 7 and 14 days, respectively.

The analysis of the photomicrographs showed in Figure 10 suggests that the treatment with A. *brachypoda* promoted the recovery of the mucous tissue, maintainment of the glands and decrease of injury border, which characterizes its protective effect. However, it is also noticed a greater extension of the sub-mucous tissue that can be associated to the neutrophil infiltrate which moves to the mucous tissue.

Figure 11 shows that the groups treated with lansoprazole and A. *brachypoda* speeded up the healing of the gastric mucous when compared to the saline group, which presents great neutrophil infiltrate in the mucous and sub-mucous layers. The images show intense reduction of the injury in the experimental groups, showing the improvement promoted by the administration of lansoprazole and A. *brachypoda.*

### - Induction of ulcer by ethanol trial:

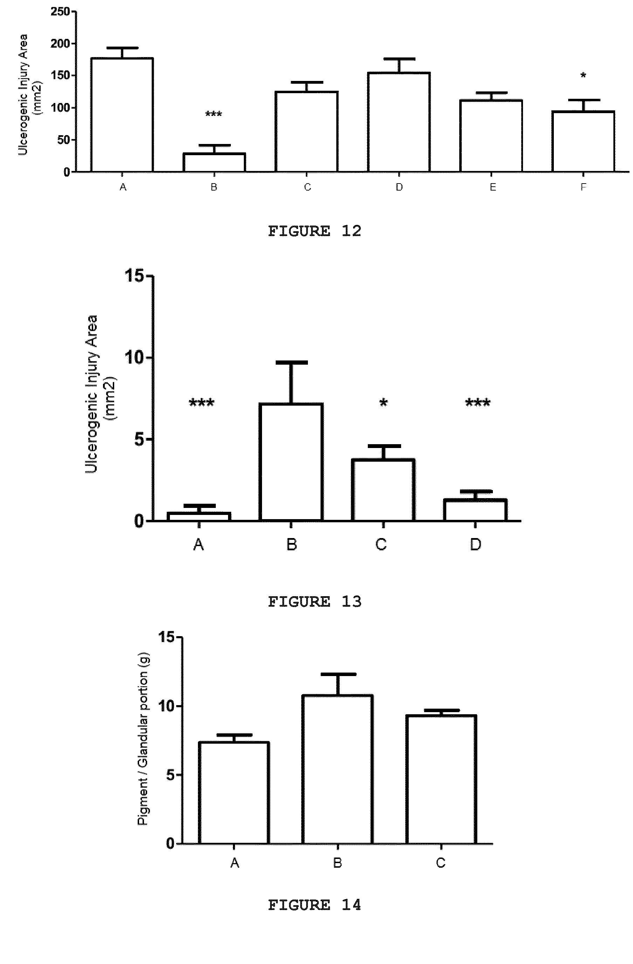

Figure 12 evinces that the anti-ulcerogenic activity of the A. *brachypoda* roots extract is the best one obtained for the dosages of 300 mg.Kg⁻¹.

The remainder dosages did not present significative activity.

In view of this result, the following trials were performed with this dosage.

### - Induction of ulcer by NSAIs trial:

According to the data showed in Figure 13, the pre-treatment with the A. *brachypoda* hydro alcoholic extract in the dosage of 300 mg.Kg⁻¹ promotes a protection of the mucous tissue in the model of ulcer induced by NSAIs.

### - Pylorus ligature and evaluation of the gastric juice parameters:

The results pointed on Table 5 below showed that the *A. brachypoda* hydro alcoholic roots extract presents antisecretory activity, since it can be noticed an increase of the pH and a decrease on the secretion volume in the group of animals treated with the extract, when compared to the group treated with saline solution.

**Table 5 - Volume (mL) and pH of the gastric acid secretion measured after the intra-duodenal administration of the A. Brachypoda hydro alcoholic extract in the pylorus ligature model**

| ***Treatment*** | ***Volume of gastric acid secretion (mL)*** | ***pH*** |
|---|---|---|
| ***Saline*** | *0.51 10* | *4.90 0.13* |
| ***Lansoprazole*** | *0.81 0.15* | *5.41 0.04**** |
| ***Cimetidine*** | *0.31 0.03*** | *5.39 0.11**** |
| ***A. brachypoda*** | 0.47 0.03 | *5.19 0.05**** |

### - Measurement of the mucus production:

The data presented in Figure 14 shows that the treatment with the A. *brachypoda* hydro alcoholic extract does not interfere of the production of mucus adhered to the mucous tissue.

### - Measurement of the MPO and measurement of the GSH levels:

The data presented on Table 6 below shows that the treatment with the A. *brachypoda* hydro alcoholic extract showed to be efficient in reducing the MPO activity.

**Table 6 - Enzymatic activity of the MPO in rats treated with the hydro-alcoholic extract (70%) of the roots of A. brachypoda plant in the model of ulcer induced by ethanol.**

| **Treatments** | **MPO (U/mg of protein)** |
|---|---|
| **Saline** | 0.26 0.01 |
| **Lansoprazole** | 0.6 0.04* |
| **A. *brachypoda*** | 0.12 0.01** |

## Claims

1. Use of compounds obtained from *Arrabidaea brachypoda* extracts **characterized by** the fact that it is to prepare a pharmaceutical composition to treat ulcerogenic diseases.

2. Use, according to claim 1, **characterized by** the fact that the ulcerogenic diseases are selected from the group which consists of ulcers which attack the skin, mucous ulcers, serous ulcers and complex ulcers.
